# EUROPEAN PATENT SPECIFICATION

(11) **EP 3 746 161 B1**
(45) Date of publication and mention of the grant of the patent: **05.07.2023**
(21) Application number: 19746902.6
(22) Date of filing: 23.01.2019
(51) Int. Cl.: A61M 15/00, G06M 1/04, G06M 1/08, G06M 1/24

(54) **INHALER DOSE COUNTER**
DOSIERZÄHLER EINES INHALATORS
COMPTEUR DE DOSES POUR INHALATEUR

(30) Priority: 31.01.2018 IN 201823001493
(43) Date of publication of application: 09.12.2020
(73) Proprietor: NEWTEC PRO MANUFACTURING PRIVATE LIMITED, Mumbai - Maharashtra 400059 (IN)
(72) Inventor: PANJABI, Akshay, Mumbai - Maharashtra 400052 (IN); PANJABI, Nirmal, Mumbai - Maharashtra 400052 (IN)
(74) Representative: Linage González, Rafael
(86) International application number: PCT/IB2019/050547
(87) International publication number: WO 2019/150228

(56) References cited:
- WO-A1-2006/062448
- WO-A1-2012/150427
- GB-A- 2 498 746
- US-A1- 2014 109 904
- US-A1- 2017 157 345
- US-A1- 2017 157 345
- US-B2- 7 100 530

## Description

### FIELD OF INVENTION

The present invention relates to mechanisms for counting and displaying the quantity of medicine/dosage remaining within an inhaler.

### DEFINITIONS

The term 'pads' used in the context of this invention refers to, but is not limited to, flanges extending from a surface.

The term 'inhaler/s' used in the context of this invention refers to, but is not limited to, Metered Dose Inhaler/s that are used by patients to self-administer aerosolized medicine via inhalation.

These definitions are in addition to those used in the art.

### BACKGROUND

Inhalers are medical devices used for delivering an aerosolized medicine via the throat into the lungs of a patient suffering from respiratory disorders such as chronic obstructive pulmonary disease (COPD), emphysema, bronchitis, asthma, and the like. Inhalers typically include a pressurized canister containing the aerosolized medicine. To release the medicine a user/patient places the inhaler in his/her mouth and depresses the canister that moves intermittently to dispense a discrete amount of the medicine in aerosol form from the inhaler. The medicine is inhaled in the lungs and reaches different airways in the lungs to provide relief to the patient. Patients suffering from chronic respiratory disorders keep such inhaler with them all the time and regularly use the inhaler whenever they face difficulty in breathing which can occur when they are in crowded places, while travelling in crowded vehicles, on dusty roads, and the like. Regular use of the inhaler can lead to fast consumption of the medicine within the canister, whereby unavailability of the medicine in the inhaler at any time when a patient suffers from breathing difficulty or an asthma attack can lead to disastrous consequences for the patient.

Hence, it is imperative for a regular user/patient of the inhaler to know the amount of medicine remaining within the canister of the inhaler. To address this requirement about the amount of medicine left in the inhaler, inhalers with counting means were devised to indicate the amount of medicine remaining in the inhaler generally over a scale.

A dose counting mechanism for indicating the quantity of dosage available within an inhaler is disclosed in document US 2017/157345.

Indian Patent IN231955 mentions a mechanical counter for a metering apparatus for metering for a medicament. The mechanical counter consists of a spindle with rotary locking, whose axis extends in parallel relationship with the axis of the metering apparatus and which is disposed in the region of the peripheral surface of the apparatus. The spindle is automatically driven by way of a transmission assembly when the metering apparatus is actuated. The number of metering portions already discharged and the number of metering portions permitted in total is quasi-continuously displayed by the mechanical counter. However, such rotary counters consist of a large number of components which make the counter complex and easily susceptible to metering errors and malfunctioning.

The devices in the prior art generally employ complex counting mechanisms which are prone to metering errors due to improper handling. Hence, there is a need for metered dose inhaler having a counting mechanism that accurately counts and indicates precise quantity of medicine remaining in the inhaler.

### OBJECTS

Some of the objects of the present invention, aimed to ameliorate one or more problems of the prior art or at least provide a useful alternative, are listed herein below.

An object of the present invention is to provide an inhaler dose counter that accurately counts the dosages of medicine available within an inhaler.

Another object of the present invention is to provide an inhaler dose counter that indicates gross and precise dosages of medicine available within an inhaler.

Another object of the present invention is to provide an inhaler dose counter that includes a simple counting mechanism that is free from counting errors.

Another object of the present invention is to provide an inhaler dose counter that can be used with a variety of canisters, valves and formulations.

Another object of the present invention is to provide an inhaler dose counter that can be used in a metered dose inhaler.

Other objects and advantages of the present invention will be more apparent from the following description which is not intended to limit the scope of the present disclosure.

### SUMMARY

In accordance with the present invention there is provided, an inhaler dose counter for indicating the quantity of dosage available within an inhaler, the inhaler having a canister with an aerosol fluid therein and an actuator including a housing body with an elongate cavity for housing the canister therein, the housing body having a top end and a bottom end and defining a vertical axis therethrough, the housing body including a mouth piece extending from the bottom end, a stem extending axially from the mouth piece into the cavity of the housing body, the stem having a sump defined therein and a passage with an orifice defined therethrough such that the stem and the sump are configured to receive a spring loaded valve of the aerosol loaded canister and to dispense a pre-measured dose of aerosol via the stem and the orifice into the mouth of a user,
- a wall of the housing body comprising,
   ▪ an outer surface defining a slit opening within the elongate cavity of the housing body;
- the inhaler dose counter comprising:
   ▪ a resilient element defined by:
      ◆ a central elongate member,
      ◆ resiliently displaceable arms extending on either side of the central elongate member,
      ◆ a key member configured at the bottom of the central elongate member, a first nose integral with the key member extending operatively inwardly from one side of the central elongate member,
         wherein in an operative configuration of the inhaler dose counter, the first nose extends through the slit into the elongate cavity of the housing body and engages with the head of the canister to be displaced within the slit in the event that the canister is depressed by the user; and
      ◆ a pusher configured below one of the arms and extending from the central elongate member, the pusher having a second pushing nose;
   ▪ a wheel formation having a ratchet wheel formation integral to the wheel formation on one operative side thereof and a first bevel gear formation integral to the wheel formation on the other operative side thereof, such that in the operative configuration of the inhaler dose counter the second pushing nose engages the ratchet wheel to angularly displace the ratchet wheel tooth by tooth in one direction;
   ▪ an elongate threaded element having a helical threaded portion, a second bevel gear formation extending from an operative lower end of the elongate threaded element and configured to engage the first bevel gear in the operative configuration of the inhaler dose counter, wherein the gear ratio between the first and second bevel gears is such that the movement of one tooth of the ratchet wheel angularly displaces both the first and second bevel gears by one tooth each, thereby causing rotational movement of the elongate threaded element including the helical threaded portion thereof by one pitch;
   ▪ a threaded formation configured to engage with the threads of the elongate threaded element, and a pointer extending from the threaded formation, the threaded formation being displaceable along the threads of the elongate threaded element in response to the rotational movement of the elongate threaded element;
**characterized by:**
- a first cover element comprising a plurality of formations formed on an inner surface of the first cover element for supporting the resilient element, the wheel formation, the elongate threaded element and the threaded formation thereon;
- the first cover with the resilient element, the wheel formation, the elongate threaded element and the threaded formation assembled therein and supported by the formations, being snap fitted on the outer surface of the wall of the housing body in the operative configuration of the inhaler dose counter; and
- a second cover element being snap fitted on the wall over the first cover element in the operative configuration of the inhaler dose counter.

The formations include:
- a first rail formation formed on the inner surface of the first cover, the rail formation supporting the threaded formation and facilitating the displacement of the threaded formation thereon;
- a first guide bracket formed on the inner surface of the first cover at a bottom portion thereof, the first guide bracket enabling the key member including a portion of the first nose to slide therein in the operative configuration of the dose counter;
- a second enclosed guide bracket formed on the inner surface of the first cover, the second enclosed guide bracket enabling an upper end of the central elongate member to slide therein in the operative configuration of the dose counter;
- a third guide bracket formed on the inner surface of the first cover at a top portion thereof, the third guide bracket accommodating a top end of the elongate threaded element and facilitating the rotational movement of the elongate threaded element in the operative configuration of the dose counter;
- a fourth guide bracket formed on the inner surface of the first cover, the fourth guide bracket accommodating a portion of the elongate threaded element and facilitating the rotational movement of the elongate threaded element in the operative configuration of the dose counter; and
- a fifth guide bracket formed on the inner surface of the first cover, the fifth guide bracket accommodating a ring formed on the elongate threaded element in the operative configuration of the dose counter;
- a first pad formed on the inner surface of the first cover and a top surface of the fifth guide bracket forming a second pad, the first and second pads facilitating the ends of the resiliently displaceable arms to rest thereon and further to resiliently deform and slide thereon in the operative configuration of the dose counter;
- a cylindrical pin formed on the inner surface of the first cover at the bottom portion thereof, the wheel formation rotatably mounted on the cylindrical pin in the operative configuration of the dose counter;
- a pawl angularly formed in a cavity formed beneath the fifth guide bracket, the pawl engaging the ratchet wheel to prevent the reverse movement of the ratchet wheel in the operative configuration of the dose counter; and
- a hollow cylindrical formation and a support formation.

Further, in the operative configuration of the inhaler dose counter,
- the support rail formation formed on the outer surface of the wall is correspondingly spaced apart from the first rail formation formed on the inner surface of the first cover and partially covers the helical threaded portion of the elongate threaded element;
- the support guide bracket formed on the outer surface of the wall is correspondingly spaced apart from the fifth guide bracket formed on the inner surface of the first cover, such that the support guide bracket and the fifth guide bracket together enclose the ring therein; and
- the protruding fixtures formed on the outer surface of the wall are accommodated in the hollow cylindrical formation and the support formation respectively.

Generally, the wall is a front wall of the housing body.

Typically, inner surface is on a back side of the first cover element.

Further, in the operative configuration of the dose counter,
- the ratchet wheel formation is proximal to the inner surface of the first cover and engages the second pushing nose when the wheel formation is mounted on the cylindrical pin, and
- the first bevel gear is distal from the inner surface of the first cover when the wheel formation is mounted on the cylindrical pin and engages the second bevel gear of the elongate threaded element, the first bevel gear being proximal to the outer surface of the wall.

Typically, the ring is formed adjacent the lower end of the elongate threaded element.

Typically, the first cover element is transparent and the second cover element includes a window to view the movement of the pointer and a linear scale adjacent to the window to indicate number of doses of aerosol fluid available in the canister corresponding to a position of the pointer.

Typically, the second cover element includes brackets formed on a back side thereof to give support thereto on the outer surface of the wall of the housing body when the second cover element is snap fitted on the wall over the first cover element.

### BRIEF DESCRIPTION OF ACCOMPANYING DRAWINGS

The present invention will now be elaborated with the help of the accompanying drawings, in which:
Figure 1a illustrates a perspective view of a housing body of an inhaler in accordance with the present invention;
Figure 1b illustrates a front view of the housing body of figure 1a;
Figure 1c illustrates a top view of the housing body of figure 1a;
Figures 2a, 2b, 2c and 2d illustrate a perspective view, a front view, a side view and a top view respectively, of a resilient element of the inhaler dose counter in accordance with the present invention;
Figures 3a, 3b, 3c and 3d illustrate a perspective view, a front view, a top view and a side view and of a wheel element of the inhaler dose counter in accordance with the present invention;
Figures 4a, 4b, 4c and 4d illustrate a perspective view, a front view, a side view and a top view of an elongate element of the inhaler dose counter in accordance with the present invention;
Figures 5a, 5b, 5c and 5d illustrate a perspective view, a front view, a side view and a top view of an indicator element of the inhaler dose counter in accordance with the present invention;
Figures 6a, 6b, 6c and 6d illustrate a perspective view, a front view and a top view of a first cover element of the inhaler dose counter in accordance with the present invention;
Figure 7 illustrates a view of the elements of the inhaler dose counter assembled within the first cover element, in an assembled operative configuration of the inhaler dose counter, in accordance with the present invention; and
Figures 8a, 8b, 8c and 8d illustrate a perspective view, a front view, a back view and a top view of a second cover element of the inhaler dose counter in accordance with the present invention.

### DETAILED DESCRIPTION

Inhalers used by patients suffering from respiratory disorders contain a specified dosage of medicine therein. Regular use of an inhaler by a patient/user results in fast consumption of the medicine. Hence, it is necessary for a patient/user to keep a track of the amount of dosage available within an inhaler to ensure that the medicine is available to him/her when desired. There have been several endeavors to create inhalers having mechanisms to indicate the dosage available in the inhalers. However, such inhalers consist of complex counting mechanisms that are prone to metering errors and are unreliable.

Hence to overcome the aforementioned problems with inhalers, the present invention envisages an inhaler dose counter for accurately counting and indicating the quantity of dosage/medicine available in an inhaler used by patients suffering from respiratory disorders.

The inhaler dose counter of the present invention will now be described with reference to the embodiments shown in the accompanying drawings. The embodiments do not limit the scope and ambit of the disclosure. The description relates purely to the examples and preferred embodiments of the disclosed method and its suggested applications.

The embodiments herein and the various features and advantageous details thereof are explained with reference to the non-limiting embodiments in the following description. Descriptions of well-known components and processing techniques are omitted so as to not unnecessarily obscure the embodiments herein. The examples used herein are intended merely to facilitate an understanding of ways in which the embodiments herein may be practiced and to further enable those of skill in the art to practice the embodiments herein. Accordingly, the examples should not be construed as limiting the scope of the embodiments herein.

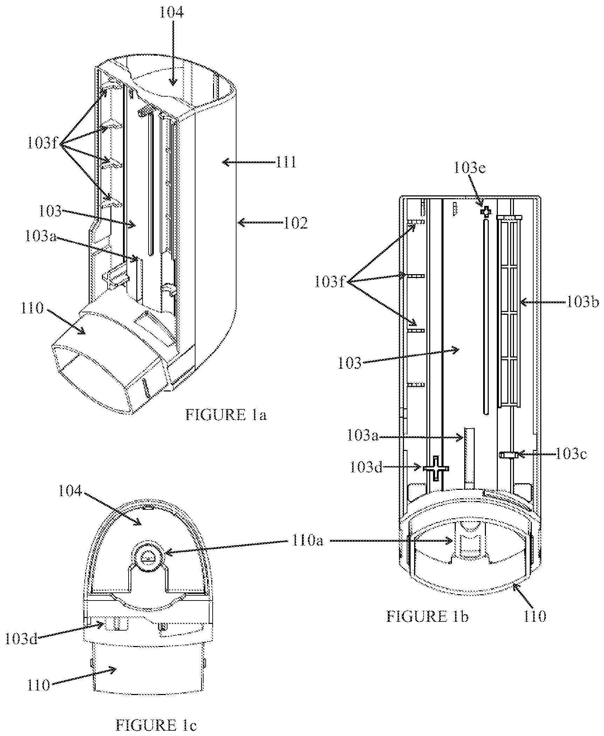

Referring to figures 1a, 1b and 1c, a perspective view, a front view and a top view respectively of a housing body of an inhaler in accordance with the present invention is illustrated. The housing body (102) comprises an elongate cavity (104) for housing a pressurized canister (not particularly shown) containing an aerosol fluid. The housing body (102) has a top end and a bottom end defining a vertical axis therethrough. The housing body (102) includes a mouth piece (110) angularly extending from the bottom end such that the axis of the mouth piece (110) is inclined at an angle to the vertical axis of the housing body.

Generally the axis of the mouth piece (110) is inclined to the vertical axis of the housing body at an angle in the range of 100 degrees to 110 degrees, and typically at an angle of 105 degrees. A stem (110a) extends axially from the mouth piece (110) into the cavity (104) of the housing body (102), such that the axis of the stem is in alignment with the vertical axis of the housing body. The stem (110a) includes a sump defined therein and a passage with an orifice defined therethrough. The canister is typically a pressurized canister comprising a spring loaded valve (not particularly shown) secured within the canister head (not particularly shown). The canister is longitudinally positioned within the housing body (102) cavity (104) such that the stem (110a) and the sump receive the spring loaded valve of the aerosol loaded canister, while a base portion of the canister juts out from the housing body (102). In order to dispense the medicine from the canister, a user/patient places the mouth piece (110) into his/her mouth and axially depresses the canister at the bottom of the base portion, whereupon a pre-measured dose of aerosol is dispensed via the stem (1 10a) and the orifice into the mouth and the throat of the user to be delivered to the lungs of the user. The housing body (102) further comprises a flat wall (103) above the mouth piece (110) and a semicircular back portion (111).

Multiple formations are moulded on the wall (103) of the housing body (102). Typically, the wall is a front wall (103) of the housing body. The outer surface of the front wall (103) of the housing body (102) comprises a slit (103a) opening within the housing body (102) cavity, a support rail formation (103b), a support guide bracket (103c), protruding fixtures (103d and 103e) and brackets (103f). The support rail formation (103b), support guide bracket (103c) and protruding fixtures (103d and 103e) are formed on the wall (103) at such locations so as to support the different elements of the inhaler dose counter.

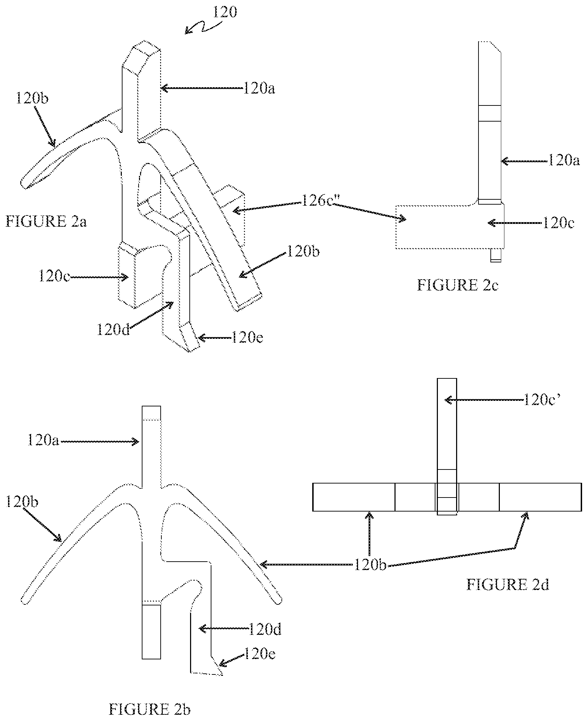

Referring to figures 2a, 2b, 2c and 2d, a perspective view, a front view, a side view and a top view respectively, of a resilient element of the inhaler dose counter in accordance with the present invention is illustrated. The element (120) has resilient elastic properties and is typically made of polyacetal. The resilient element (120) is defined by a central elongate member (120a), resiliently displaceable arms (120b), a key member (120c) including an integral first nose (120c'), and a pusher (120d) with a second pushing nose (120e). The arms (120b) are elastically deformable and extend on either side of the central elongate member (120a). The key member (120c) is integral with the central elongate member (120a). The key member (120c) is configured at the bottom of the central elongate member (120a) and the integral first nose (120c') extends operatively inwardly from one side of the central elongate member (120a). In accordance with one embodiment, the first nose (120c') of the key member (120c) extends perpendicularly from one side of the central elongate member (120a). The pusher arm (120d) is configured below one of the resiliently displaceable elliptical shaped arms (120b) and extends from a side of the central elongate member (120a).

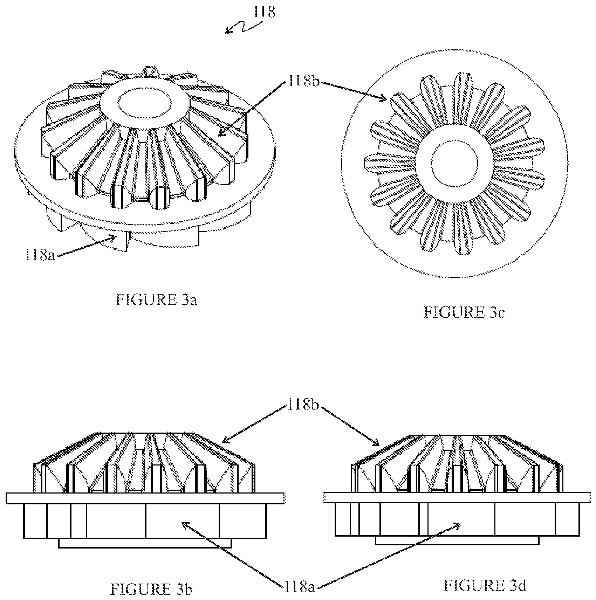

Referring to figures 3a, 3b, 3c and 3d, a perspective view, a front view, a top view and a side view respectively of a wheel element of the inhaler dose counter in accordance with the present invention is illustrated. The inhaler dose counter comprises the wheel element in the form of a wheel formation (118) having a ratchet wheel (118a) formation integral thereto on one operative side thereof and a first bevel gear (118b) formation integral thereto on the other operative side thereof.

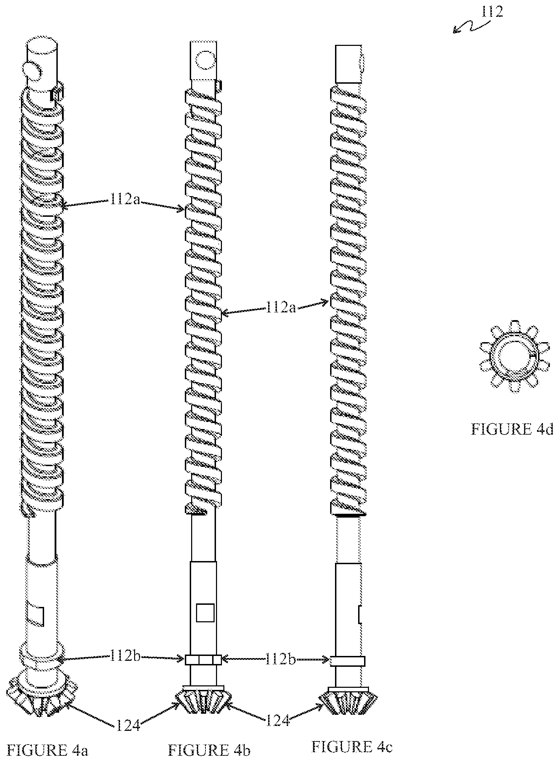

Referring to figures 4a, 4b, 4c and 4d, a perspective view, a front view, a side view and a top view respectively of an elongate element of the inhaler dose counter in accordance with the present invention is illustrated. The inhaler dose counter comprises the elongate element in the form of an elongate threaded element (112) having a plurality of helical threads (112a) and a ring (112b) formed thereon, and a second bevel gear (124) extending from an operative lower end of the elongate threaded element (112). Typically, the ring (112b) is formed adjacent the lower end of the elongate threaded element (112).

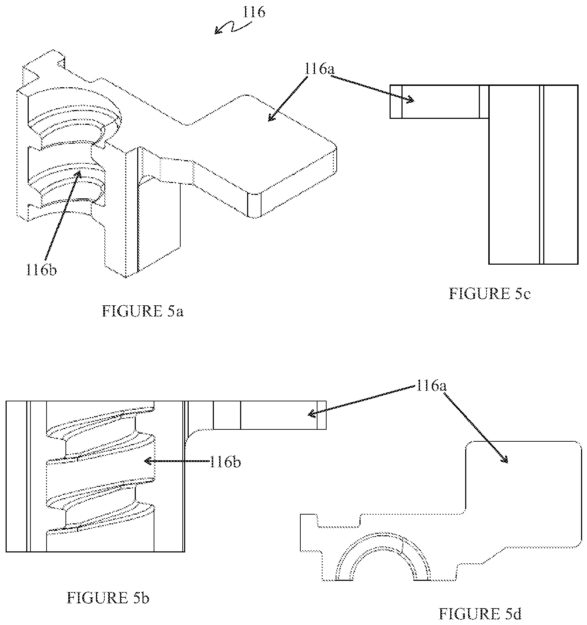

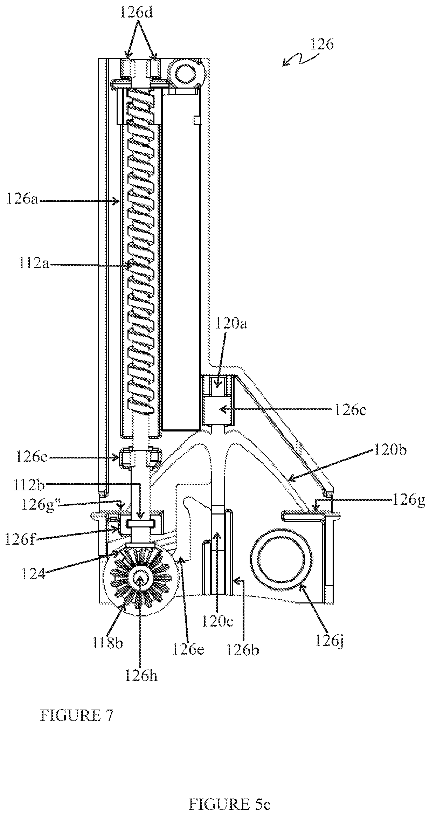

Referring to figures 5a, 5b, 5c and 5d, a perspective view, a front view, a side view and a top view of an indicator element of the inhaler dose counter in accordance with the present invention is illustrated. The inhaler dose counter comprises the indicator element in the form of a threaded formation (116) having a pointer (116a) extending therefrom and threads (116b) formed on an operative back side of the threaded formation (116).

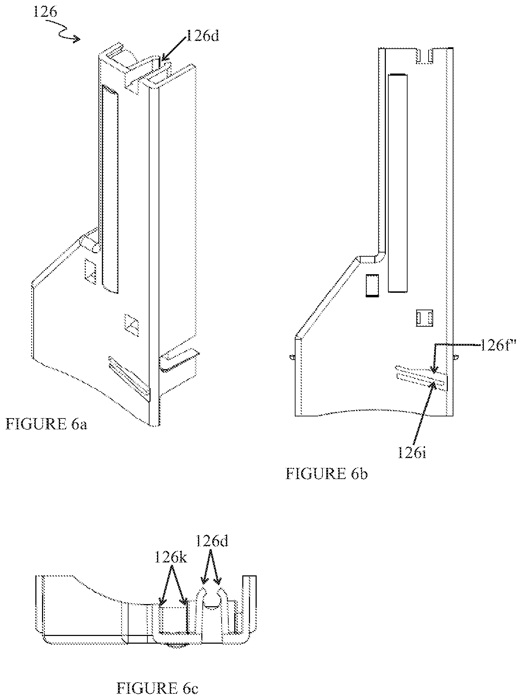

Referring to figures 6a, 6b and 6c a perspective view, a front view and a top view of a first cover element of the inhaler dose counter in accordance with the present invention is illustrated. The first cover element (126) comprises a plurality of formations formed on the inner surface of the back side of the first cover element. The formations include a first rail formation (126a), a first guide bracket (126b), a second enclosed guide bracket (126c), a third guide bracket (126d), a fourth guide bracket (126e), a fifth guide bracket (126f), a pair of pads (126g, 126g'), a cylindrical pin (126h), a pawl (126i), a hollow cylindrical formation (126j) and a support formation (126k), formed on the inner surface of the back side of the first cover element (126).

The rail formation (126a) supports the threaded formation (116) and facilitates the displacement of the threaded formation (116) thereon. The first guide bracket (126b) is formed on the inner surface of the back side of the first cover (126) at a bottom portion thereof. The first guide bracket (126b) enables the key member (120c) including a portion of the first nose (120c') to slide therein in the operative configuration of the inhaler dose counter. The second enclosed guide bracket (126c) is formed on the inner surface of the back side of the first cover (126). The second enclosed guide bracket (126c) enables an upper end of the central elongate member (120a) to slide therein in the operative configuration of the inhaler dose counter. The third guide bracket (126d) is formed on the inner surface of the back side of the first cover (126) at a top portion thereof. The third guide bracket (126d) accommodates a top end of the elongate threaded element (112) and facilitates the rotational movement of the elongate threaded element (112) in the operative configuration of the inhaler dose counter. The fourth guide bracket (126e) is formed on the inner surface of the back side of the first cover (126). The fourth guide bracket (126e) accommodates a portion of the elongate threaded element (112) and facilitates the rotational movement of the elongate threaded element (112) in the operative configuration of the inhaler dose counter. The fifth guide bracket (126f) is formed on the inner surface of the back side of the first cover (126). The fifth guide bracket (126f) accommodates the ring (112b) formed adjacent the lower end of the elongate threaded element (112) and facilitates the rotational movement thereof in the operative configuration of the inhaler dose counter. The pair of pads includes a first pad (126g) formed on the inner surface of the back side of the first cover (126) and a top surface of the fifth guide bracket (126f) forming a second pad (126g'). The first and second pads (126g, 126g') facilitate the ends of the resiliently displaceable arms (120b) to rest thereon and further to resiliently deform and slide thereon in the operative configuration of the inhaler dose counter. The cylindrical pin (126h) is formed on the inner surface of the back side of the first cover (126) at the bottom portion thereof. The wheel formation (118) is rotatably mounted on the cylindrical pin (126h), such that the ratchet wheel formation (118a) is proximal to the inner surface of the back side of the first cover (126) and engages the second pushing nose (120e) in the operative configuration of the inhaler dose counter, while the first bevel gear (118b) is distal from the inner surface of the back side of the first cover (126). The pawl (126i) is angularly formed in a cavity (126f) formed beneath the fifth guide bracket (126f). The pawl (126i) engages the teeth of the ratchet wheel (118a) to prevent the reverse movement of the ratchet wheel in the operative configuration of the inhaler dose counter. The hollow cylindrical formation (126j) is formed on the inner surface of the back side of the first cover (126) at a bottom portion thereof, and the support formation (126k) is formed on the inner surface of the back side of the first cover (126) at a top portion thereof. The hollow cylindrical formation (126j) and the support formation (126k) accommodate the protruding fixtures (103d and 103e) formed on the outer surface of the front wall (103) of the housing body (102).

Referring to figure 7, the elements of the inhaler dose counter assembled within the first cover element, in an assembled operative configuration of the inhaler dose counter, in accordance with the present invention is illustrated. The resilient element (120), wheel formation (118), elongate threaded element (112) and threaded formation (116) are assembled on the inner surface of the back side of the first cover element (126). In order to assemble the elements in the first cover (126), the resilient element (120) is placed on the inner surface of the back side of the first cover such that the key member (120c) and a portion of the first nose (120c') thereof is slidably accommodated in the first guide bracket (126b) and the upper end of the central elongate member (120a) is slidably accommodated in the second enclosed guide bracket (126c). Next, the wheel formation (118) is mounted on the cylindrical pin (126h) with the ratchet wheel (118a) proximal to the inner surface of the back side of the first cover (126) and the first bevel gear (118b) distal therefrom. Next, the front side of the threaded formation (116) is placed on the rail formation (126a). Finally, the elongated threaded element (112) is placed on the inner surface of the back side of the first cover such that the threaded portion (112a) thereof engages with the threads (116b) on the operative back side of the threaded formation (116), the top portion thereof is accommodated in a snap-fit manner in the third guide bracket (120d), at least a non-threaded portion thereof is accommodated in a snap-fit manner in the fourth guide bracket (120e), the ring (112b) is accommodated in the fifth guide bracket (126f) and the second bevel gear (124) extending from the operative lower end thereof engages with the first bevel gear (118b) of the wheel formation (118) mounted on the cylindrical pin (126h). The first cover element (126) with the elements (120, 118, 112, 116) assembled therein is then snap fitted on the wall (103) of the housing body (102) of the inhaler. In accordance with one embodiment, the first cover element (126) is transparent.

In accordance with the operative configuration of the inhaler dose counter, upon snap fitting the first cover element (126) with the elements (120, 118, 112, 116) assembled therein on the wall (103), the operatively inwardly extending first nose (120c') of the key member (120c) extends through the slit (103a) into the elongate cavity of the housing body (102) and engages with the head of the canister (104); the wheel formation (118) is positioned on the cylindrical pin (126h) with the ratchet wheel (118a) proximal to the inner surface of the back side of the first cover (126) and the first bevel gear (118c) distal from the inner surface of the back side of the first cover (126) but proximal to the outer surface of the wall (103); the support rail formation (103b) formed on the outer surface of the wall (103) is correspondingly spaced apart from the rail formation (126a) formed on the inner surface of the back side of the first cover (126), and partially covers the helical threaded portion (112a) of the elongate threaded element; the support guide bracket (103c) formed on the outer surface of the wall (103) is correspondingly spaced apart from the fifth guide bracket (126f) on the inner surface of the first cover (126), and partially covers the ring (112b) formed adjacent the lower end of the elongate threaded element (112) such that the support guide bracket (103c) and the fifth guide bracket (126f) together enclose the ring (112b) therein. The hollow cylindrical formation (126j) and the support formation (126k) accommodate therein the protruding fixtures (103d and 103e) formed on the outer surface of the front wall (103) of the housing body (102).

In the event that the user axially depresses the canister (104) at the bottom of the base portion, the head of the canister (104) engaging the first nose (120c') of the key member (120c) causes the key member (120c) to slide down within the slit (103a) along with the displacement of the canister in the housing (102) cavity, thereby causing displacement of the resilient element (120). The upper end of the central elongate member (120a) slides in the second enclosed guide bracket (126c), and the ends of the flexible arms (120b) slide on the pads (103g). The second pushing nose (120e) engages the teeth of the ratchet wheel (118a) to angularly displace the ratchet wheel (118a) by one tooth at a time. The second bevel gear (124) of the elongate threaded element (112) engages with the first bevel gear (118b) of the wheel formation (118), wherein the gear ratio between the first (118b) and second (124) bevel gears is such that the movement of one tooth of the ratchet wheel (118a) angularly displaces both the first (118b) and second (124) bevel gears by one tooth each, thereby causing rotational movement of the elongate threaded element (112). The threads (116b) of the threaded formation (116) engage with the helical threads (112a) of the elongate threaded element (112), causing the threaded formation (116) and thereby the pointer (116a) to displace down along the elongate element (112) in response to the rotational movement of the elongate element (112), thereby counting down the dosage available in the canister (104) by one count. Each such depression of the canister (104) causes decrease of the dosage available in the canister (104) by one count. A precise number of teeth are formed on the ratchet wheel (118a), the first bevel gear (118b) and the second bevel gear (124) and a precise number of helical threads are formed on the elongate threaded element (112) to count down a specific number of doses. In accordance with one embodiment, the number of doses to be counted down is 200 doses; in accordance with another embodiment, the number of doses to be counted down is 120 doses; and so on.

After the aerosol fluid is dispensed from the canister, the spring loaded valve in the canister (104) relaxes causing the canister to move back to its original position. The resilient elastic properties of the flexible arms (120b) results in resetting motion of the arms (120b) causing the arms (120b) to move upwards, whereby the key member (120c) slides back up in the slit (103a) and the ends of the arms (120b) slide back on the pads (103b) and come to rest and are ready for subsequent use for release of next dosage. The pusher (120d) also moves upwards against the ratchet wheel (118a) on the wheel formation (118). However, the reverse movement of the ratchet wheel (118a) and the wheel formation (118) is restricted by the pawl (103i) angularly formed in the cavity (126f) formed beneath the fifth guide bracket (126f).

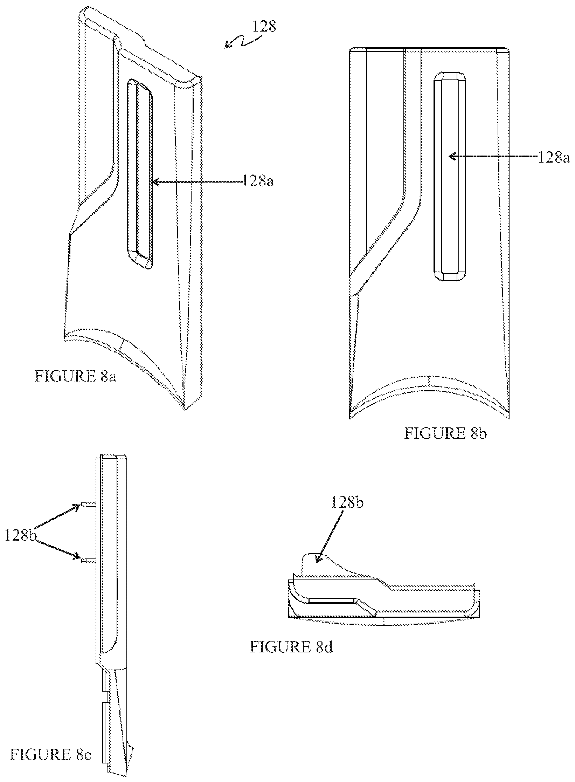

Referring to figures 8a, 8b, 8c and 8d, a perspective view, a front view, a side view and a top view of a second cover element of the inhaler dose counter in accordance with the present invention is illustrated. The second cover element (128) is snap fitted on the wall (103) over the first cover element (126). The second cover element (128) includes a window (128a) to view the pointer (116a) movement and a scale adjacent the window (128a) to indicate a quantity of aerosol fluid available in the canister corresponding to a position of the pointer (116a). In accordance with one embodiment, the scale is printed adjacent the window (128a). In accordance with another embodiment, the scale is in the form of a sticker pasted adjacent the window (128a). In accordance with yet another embodiment, the scale is engraved adjacent the window (128a). In accordance with still another embodiment, the scale is embossed adjacent the window (128a). As described herein above, the indicator element comprising the threaded formation (116) and thereby the pointer (116a) travels from the top of the window (128a) to the bottom of window (128a) with the usage of the inhaler (100).

With every single use indicating element comprising the threaded formation (116) and thereby the pointer (116a) travels down by a predefined distance indicating the quantity of aerosol fluid remaining in the inhaler on the scale. A bottom position of the pointer (116a) indicates that the medicine in the inhaler is exhausted. Typically, the scale includes color variations to indicate different quantity of dosage available in the canister. In accordance with an embodiment, the pointer (116a) is composed of luminescent materials causing the pointer to glow in dark and enabling user to view the quantity of dosage available even in no light conditions. The second cover element further includes brackets (128b) formed on a back side thereof. The brackets (128b) give support to the second cover element on the outer surface of the wall (103) of the housing body (102) when it is snap fitted on the wall (103) over the first cover element (126).

Typically, the second cover element (128) is sized such that the length of the second cover element (128) complements the length of the elongate bodies of different sizes of canisters.

Thus, the combination of the wall (103), the first cover element (126) with the specially moulded formations (126a-126k) for supporting the resilient element (120), the wheel element comprising wheel formation (118), the elongate threaded element (112), the indicator element comprising the threaded formation (116), and the second cover element (128) provide an inhaler dose counter with a simple mechanism that is error free and accurately counts and indicates the quantity of dosage available in an inhaler, enabling the inhaler to be conveniently used by patients suffering from respiratory disorders.

### TECHNICAL ADVANCEMENTS AND ECONOMIC SIGNIFICANCE

The technical advancements offered by the inhaler dose counter of the present invention include the realization of:
- accurate counting of the quantity of medicine available within an inhaler;
- indicating a precise quantity of medicine available within an inhaler;
- a simple counting mechanism that is free from counting errors; and
- usability with a variety of canisters, valves and formulations.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

The use of the expression "at least" or "at least one" suggests the use of one or more elements or ingredients or quantities, as the use may be in the embodiment of the invention to achieve one or more of the desired objects or results.

Any discussion of documents, acts, materials, devices, articles or the like that has been included in this specification is solely for the purpose of providing a context for the invention. It is not to be taken as an admission that any or all of these matters form part of the prior art base or were common general knowledge in the field relevant to the invention as it existed anywhere before the priority date of this application.

The numerical values mentioned for the various physical parameters, dimensions or quantities are only approximations and it is envisaged that the values higher/lower than the numerical values assigned to the parameters, dimensions or quantities fall within the scope of the invention, unless there is a statement in the specification specific to the contrary.

The foregoing description of the specific embodiments will so fully reveal the general nature of the embodiments herein that others can, by applying current knowledge, readily modify and/or adapt for various applications such specific embodiments without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation.

The invention is defined by the following claims.

## Claims

1. An inhaler dose counter for indicating the quantity of dosage available within an inhaler (100), the inhaler having a canister with an aerosol fluid therein and an actuator including a housing body (102) with an elongate cavity (104) for housing the canister therein, the housing body having a top end and a bottom end and defining a vertical axis therethrough, the housing body including a mouth piece (110) extending from the bottom end, a stem (110a) extending axially from the mouth piece into the cavity (104) of the housing body, the stem having a sump defined therein and a passage with an orifice defined therethrough such that the stem and the sump are configured to receive a spring loaded valve of the aerosol loaded canister and to dispense a pre-measured dose of aerosol via the stem and the orifice into the mouth of a user, a wall (103) of the housing body (102) comprising an outer surface defining a slit (103a) opening within the elongate cavity (104) of the housing body;
said inhaler dose counter comprising:
▪ a resilient element (120) defined by:
◆ a central elongate member (120a),
◆ resiliently displaceable arms (120b) extending on either side of the central elongate member (120a),
◆ a key member (120c) configured at the bottom of said central elongate member (120a), a first nose (120c') integral with said key member extending operatively inwardly from one side of said central elongate member,
wherein in an operative configuration of said inhaler dose counter, said first nose (120c') extends through said slit (103a) into the elongate cavity (104) of the housing body (102) and engages with the head of the canister to be displaced within said slit in the event that the canister is depressed by the user; and
◆ a pusher (120d) configured below one of said arms (120b) and extending from said central elongate member (120a), said pusher having a second pushing nose (120e);
▪ a wheel formation (118) having a ratchet wheel formation (118a) integral to said wheel formation on one operative side thereof and a first bevel gear formation (118b) integral to said wheel formation on the other operative side thereof, such that in the operative configuration of said inhaler dose counter said second pushing nose (120e) engages said ratchet wheel (118a) to angularly displace said ratchet wheel tooth by tooth in one direction;
▪ an elongate threaded element (112) having a helical threaded portion (112a), a second bevel gear formation (124) extending from an operative lower end of said elongate threaded element and configured to engage said first bevel gear (118b) in the operative configuration of said inhaler dose counter, wherein the gear ratio between said first and second bevel gears (118b, 124) is such that the movement of one tooth of said ratchet wheel (118a) angularly displaces both said first and second bevel gears (118b, 124) by one tooth each, thereby causing rotational movement of said elongate threaded element (112) including the helical threaded portion (112a) thereof by one pitch;
▪ a threaded formation (116) configured to engage with the threads (112a) of said elongate threaded element, and a pointer (116a) extending from said threaded formation, said threaded formation being displaceable along the threads of said elongate threaded element in response to the rotational movement of said elongate threaded element;
**characterized by:**
• a first cover element (126) comprising a plurality of formations formed on an inner surface of said first cover element, said formations supporting said resilient element (120), said wheel formation (118), said elongate threaded element (112) and said threaded formation (116) thereon;
• said first cover (126) with said resilient element (120), said wheel formation (118), said elongate threaded element (112) and said threaded formation (116) assembled therein and supported by said formations, being snap fitted on said outer surface of said wall (103) of said housing body (102) in the operative configuration of said inhaler dose counter; and
• a second cover element (128) being snap fitted on said wall (103) over said first cover element (126) in the operative configuration of said inhaler dose counter;
wherein said formations include:
o a first rail formation (126a) formed on said inner surface of said first cover (126), said rail formation supporting said threaded formation (116) and facilitating the displacement of said threaded formation thereon;
o a first guide bracket (126b) formed on said inner surface of said first cover (126) at a bottom portion thereof, said first guide bracket enabling said key member (120c) including a portion of said first nose (120c') to slide therein in the operative configuration of said dose counter;
o a second enclosed guide bracket (126c) formed on said inner surface of said first cover (126), said second enclosed guide bracket enabling an upper end of said central elongate member (120a) to slide therein in the operative configuration of said dose counter;
o a third guide bracket (126d) formed on said inner surface of said first cover (126) at a top portion thereof, said third guide bracket accommodating a top end of said elongate threaded element (112) and facilitating the rotational movement of said elongate threaded element in the operative configuration of said dose counter;
o a fourth guide bracket (126e) formed on said inner surface of said first cover (126), said fourth guide bracket accommodating a portion of said elongate threaded element (112) and facilitating the rotational movement of said elongate threaded element in the operative configuration of said dose counter; and
o a fifth guide bracket (126f) formed on said inner surface of said first cover (126), said fifth guide bracket accommodating a ring (112b) formed on said elongate threaded element (112) in the operative configuration of said dose counter;
o a first pad (126g) formed on said inner surface of said first cover (126) and a top surface of said fifth guide bracket (126f) forming a second pad (126g'), said first and second pads facilitating the ends of said resiliently displaceable arms (120b) to rest thereon and further to resiliently deform and slide thereon in the operative configuration of said dose counter;
o a cylindrical pin (126h) formed on said inner surface of said first cover (126) at the bottom portion thereof, said wheel formation (118) rotatably mounted on said cylindrical pin in the operative configuration of said dose counter;
o a pawl (126i) angularly formed in a cavity (126f) formed beneath said fifth guide bracket (126f), said pawl engaging said ratchet wheel (118a) to prevent the reverse movement of said ratchet wheel in the operative configuration of said dose counter; and
o a hollow cylindrical formation (126j) and a support formation (126k).

2. The inhaler dose counter as claimed in claim 1, wherein said wall (103) of said housing body (102) includes a support rail formation (103b), a support guide bracket (103c) and a plurality of protruding fixtures (103d, 103e) and brackets (103f) formed on said outer surface of said wall.

3. The inhaler dose counter as claimed in claim 2, wherein, in the operative configuration of the inhaler dose counter,
• said support rail formation (103b) formed on said outer surface of said wall (103) is correspondingly spaced apart from said first rail formation (126a) formed on said inner surface of said first cover (126) and partially covers said helical threaded portion (112a) of said elongate threaded element (112);
• said support guide bracket (103c) formed on said outer surface of said wall (103) is correspondingly spaced apart from said fifth guide bracket (126f) formed on said inner surface of the first cover (126), such that said support guide bracket (103c) and said fifth guide bracket (126f) together enclose said ring (112b) therein; and
• said protruding fixtures (103d, 103e) formed on said outer surface of said wall (103) are accommodated in said hollow cylindrical formation (126j) and the support formation (126k) respectively.

4. The inhaler dose counter as claimed in claim 1, wherein said wall (103) is a front wall of said housing body (102).

5. The inhaler dose counter as claimed in claim 1, wherein said inner surface is on a back side of said first cover element (126).

6. The inhaler dose counter as claimed in claim 1, wherein, in the operative configuration of said dose counter,
• said ratchet wheel formation (118a) is proximal to said inner surface of said first cover (126) and engages said second pushing nose (126e) when said wheel formation (118) is mounted on said cylindrical pin (126h), and
• said first bevel gear (118b) is distal from said inner surface of said first cover (126) when said wheel formation (118) is mounted on said cylindrical pin (126h) and engages said second bevel gear (124) of said elongate threaded element (112), said first bevel gear (118b) being proximal to said outer surface of said wall (103).

7. The inhaler dose counter as claimed in claim 1, wherein said ring (112b) is formed adjacent said lower end of said elongate threaded element (112).

8. The inhaler dose counter as claimed in claim 1, wherein said first cover element (126) is transparent and said second cover element (128) includes a window (128a) to view the movement of said pointer (116a) and a linear scale adjacent to said window to indicate number of doses of aerosol fluid available in the canister corresponding to a position of said pointer.

9. The inhaler dose counter as claimed in claim 1, wherein said second cover element (128) includes brackets (128b) formed on a back side thereof to give support thereto on said outer surface of said wall (103) of said housing body (102) when said second cover element is snap fitted on said wall over said first cover element (126).

## Patentansprüche

1. Dosiszähler für Inhalatoren zur Angabe der Menge der verfügbaren Dosen innerhalb eines Inhalators (100), wobei der Inhalator einen Behälter mit einer Aerosolflüssigkeit darin sowie einen Auslöser einschließlich eines Gehäusekörpers (102) mit einem länglichen Hohlraum (104) zur Aufnahme des Behälters darin aufweist, wobei der Gehäusekörper ein oberes Ende und ein unteres Ende aufweist und eine vertikale Achse durch dieselben definiert, wobei der Gehäusekörper ein Mundstück (110), das sich vom unteren Ende aus erstreckt, einen Schaft (110a), der sich axial vom Mundstück in den Hohlraum (104) des Gehäusekörpers erstreckt, wobei der Schaft einen darin definierten Sammelbehälter aufweist, sowie einen Durchlass mit einer durch denselben definierten Düse enthält, so dass der Schaft und der Sammelbehälter so ausgelegt sind, dass sie ein federbelastetes Ventil des mit Aerosol geladenen Behälters aufnimmt und ein abgemessene Dosis des Aerosols über den Schaft und die Düse in den Mund eines Nutzers abgibt, wobei eine Wand (103) des Gehäusekörpers (102) eine Außenfläche umfasst, die einen Schlitz (103a) definiert, der sich innerhalb des länglichen Hohlraums (104) des Gehäusekörpers öffnet;
wobei der Dosiszähler des Inhalators Folgendes umfasst:
▪ ein federndes Element (120), definiert durch:
◆ ein in der Mitte gelegenes längliches Teil (120a),
◆ elastisch verschiebbare Arme (120b), die sich auf jeder Seite des in der Mitte gelegenen länglichen Teils (120a) erstrecken,
◆ ein Federteil (120c), das am Boden des in der Mitte gelegenen länglichen Teils (120a) ausgebildet ist, eine erste Nase (120c'), die in das Federteil integriert ist und sich funktionell von einer Seite des in der Mitte gelegenen länglichen Teils nach innen erstreckt und sich somit in einer funktionellen Anordnung des Dosiszählers des Inhalators befindet, wobei sich die erste Nase (120c') durch den Schlitz (103a) in den länglichen Hohlraum (104) des Gehäusekörpers (102) erstreckt und in Eingriff mit dem Kopf des Behälters kommt, um in den Schlitz verschoben zu werden, falls der Behälter durch den Nutzer zusammengedrückt wird; und
◆ einen Schieber (120d), der unterhalb eines der Arme (120b) ausgebildet ist und sich vom in der Mitte gelegenen länglichen Teil (120a) erstreckt, wobei der Schieber eine zweite Schiebenase (120e) aufweist;
▪ eine Radanordnung (118) mit einer Sperrradanordnung (118a), die auf einer funktionellen Seite derselben in die Radanordnung integriert ist, sowie eine erste Kegelradanordnung (118b), die auf der anderen funktionellen Seite derselben in die Radanordnung integriert ist, so dass in der funktionellen Anordnung des Dosiszählers des Inhalators die zweite Schiebenase (120e) in das Sperrrad (118a) eingreift, um das Sperrrad winklig Zahn um Zahn in eine Richtung zu verschieben;
▪ ein längliches Gewindeelement (112) mit einem spiralförmigen Gewindeabschnitt (112a), eine zweite Kegelradanordnung (124), die sich von einem funktionellen unteren Ende des länglichen Gewindeelements erstreckt und so ausgelegt ist, dass sie in der funktionellen Anordnung des Dosiszählers des Inhalators in das erste Kegelrad (118b) eingreift, wobei das Übersetzungsverhältnis zwischen dem ersten und dem zweiten Kegelrad (118b, 124) so ist, dass die Bewegung eines Zahns des Sperrrads (118a) sowohl das erste als auch das zweite Kegelrad (118b, 124) um jeweils einen Zahn winklig verschiebt, wodurch eine Drehbewegung des länglichen Gewindeelements (112) einschließlich des spiralförmigen Abschnitts (112a) desselben um eine Stufe bewirkt;
▪ eine Gewindeanordnung (116), die so ausgebildet ist, dass sie mit den Gewinden (112a) des länglichen Gewindeelements in Eingriff steht, sowie ein Zeiger (116a), der sich von der Gewindeanordnung erstreckt, wobei die Gewindeanordnung entlang der Gewinde des länglichen Gewindeelements in Reaktion auf die Drehbewegung des länglichen Gewindeelements verschiebbar ist;
**gekennzeichnet durch:**
• ein erstes Abdeckelement (126), das eine Vielzahl von Anordnungen umfasst, die auf einer Innenfläche des ersten Abdeckelements ausgebildet sind, wobei die Anordnungen das federnde Element (120), die Radanordnung (118), das längliche Gewindeelement (112) und die Gewindeanordnung (116) darauf stützen;
• **dadurch, dass** das erste Abdeckelement (126) mit dem federnden Element (120), der Radanordnung (118), dem länglichen Gewindeelement (112) und der Gewindeanordnung (116), die darin montiert sind und durch die Anordnungen gestützt wird, auf der Außenfläche der Wand (103) des Gehäusekörpers (102) in der funktionellen Anordnung des Dosiszählers des Inhalators einrastet; und
• ein zweites Abdeckelement (128), das auf der Wand (103) über dem ersten Abdeckelement (126) in der funktionellen Anordnung des Dosiszählers des Inhalators einrastet;
wobei die Anordnungen Folgendes enthalten:
o eine erste Schienenanordnung (126a), die auf der Innenfläche der ersten Abdeckung (126) ausgebildet ist, wobei die Schienenanordnung die Gewindeanordnung (116) stützt und die Verschiebung der Gewindeanordnung auf derselben ermöglicht;
o einen ersten Führungsbügel (126b), der auf der Innenfläche der ersten Abdeckung (126) an einem unteren Abschnitt derselben ausgebildet ist, wobei der erste Führungsbügel ermöglicht, dass das Federteil (120c) einschließlich eines Abschnitts der ersten Nase (120c') in der funktionellen Anordnung des Dosiszählers in demselben gleitet;
o einen zweiten eingeschlossenen Führungsbügel (126c), der auf der Innenfläche der ersten Abdeckung (126) ausgebildet ist, wobei es der zweite eingeschlossene Führungsbügel ermöglicht, dass ein oberes Ende des in der Mitte gelegenen länglichen Teils (120a) in der funktionellen Anordnung des Dosiszählers in demselben gleitet;
o einen dritten Führungsbügel (126d), der auf der Innenfläche der ersten Abdeckung (126) in einem obersten Abschnitt derselben ausgebildet ist, wobei der dritte Führungsbügel ein oberstes Ende des länglichen Gewindeelements (112) aufnimmt und die Drehbewegung des länglichen Gewindeelements in der funktionellen Anordnung des Dosiszählers ermöglicht;
o einen vierten Führungsbügel (126e), der auf der Innenfläche der ersten Abdeckung (126) ausgebildet ist, wobei der vierte Führungsbügel einen Abschnitt des länglichen Gewindeelements (112) aufnimmt und die Drehbewegung des länglichen Gewindeelements in der funktionellen Anordnung des Dosiszählers ermöglicht; und
o einen fünften Führungsbügel (126f), der auf der Innenfläche der ersten Abdeckung (126) ausgebildet ist, wobei der fünfte Führungsbügel einen Ring (112b) aufnimmt, der in der funktionellen Anordnung des Dosiszählers auf dem länglichen Gewindeelement (112) ausgebildet ist;
o ein erstes Pad (126g), das auf der Innenfläche der ersten Abdeckung (126) ausgebildet ist, und eine oberste Fläche des fünften Führungsbügels (126f), die ein zweites Pad (126g`) ausbildet, wobei das erste und das zweite Pad ermöglichen, dass die Enden der elastisch verschiebbaren Arme (120b) darauf ruhen und sich ferner in der funktionellen Anordnung des Dosiszählers elastisch verformen und darauf gleiten;
o ein zylindrischer Stift (126h), der auf der Innenfläche der ersten Abdeckung (126) am unteren Abschnitt derselben ausgebildet ist, wobei die Radanordnung (118) in der funktionellen Anordnung des Dosiszählers drehbar auf dem zylindrischen Stift montiert ist;
o eine Sperrklinke (126i), die winklig in einem Hohlraum (126f) ausgebildet ist, der unterhalb des fünften Führungsbügels (126f) ausgebildet ist, wobei die Sperrklinke in das Sperrrad (118a) eingreift, um in der funktionellen Anordnung des Dosiszählers die Rückwärtsbewegung des Sperrrads zu verhindern; und
∘ eine hohlzylindrische Anordnung (126j) sowie eine Stützanordnung (126k).

2. Dosiszähler für Inhalatoren nach Anspruch 1, wobei die Wand (103) des Gehäusekörpers (102) eine Stützschienenanordnung (103b), einen Stützführungsbügel (103c) und eine Vielzahl hervorstehender Vorrichtungen (103d, 103e) sowie Bügel (103f) enthält, die auf der Außenfläche der Wand ausgebildet sind.

3. Dosiszähler für Inhalatoren nach Anspruch 2, wobei in der funktionellen Anordnung des Dosiszählers
• die Stützschienenanordnung (103b), die auf der Außenfläche der Wand (103) ausgebildet ist, entsprechend von der ersten Schienenanordnung (126a) beabstandet ist, die auf der Innenfläche der ersten Abdeckung (126) ausgebildet ist, und teilweise den spiralförmigen Gewindeabschnitt (112a) des länglichen Gewindeelements (112) abdeckt;
• wobei der Stützführungsbügel (103c), der auf der Außenfläche der Wand (103) ausgebildet ist, entsprechend vom fünften Führungsbügel (126f) beabstandet ist, der auf der Innenfläche der ersten Abdeckung (126) ausgebildet ist, wobei der Stützführungsbügel (103c) und der fünfte Führungsbügel (126f) gemeinsam den Ring (112b) darin einschließen; und
• wobei die hervorstehenden Vorrichtungen (103d, 103e), die auf der Außenfläche der Wand (103) ausgebildet sind, in der hohlzylindrischen Anordnung (126j) bzw. der Stützanordnung (126k) aufgenommen sind.

4. Dosiszähler für Inhalatoren nach Anspruch 1, wobei die Wand (103) eine Vorderwand des Gehäusekörpers (102) ist.

5. Dosiszähler für Inhalatoren nach Anspruch 1, wobei sich die Innenfläche auf der Rückseite des ersten Abdeckelements (126) befindet.

6. Dosiszähler für Inhalatoren nach Anspruch 1, wobei sich in der funktionellen Anordnung des Dosiszählers
• die Sperrradanordnung (118a) in der Nähe der Innenfläche der ersten Abdeckung (126) befindet und im Eingriff mit der zweiten Schiebenase (126e) steht, wenn die Radanordnung (118) auf dem zylindrischen Stift (126h) montiert ist, und
• sich das erste Kegelrad (118b) entfernt von der Innenfläche der ersten Abdeckung (126) befindet, wenn die Radanordnung (118) auf dem zylindrischen Stift (126h) montiert ist und im Eingriff mit dem zweiten Kegelrad (124) des länglichen Gewindeelements (112) steht, wobei sich das erste Kegelrad (118b) in der Nähe der Außenfläche der Wand (103) befindet.

7. Dosiszähler für Inhalatoren nach Anspruch 1, wobei der Ring (112b) angrenzend an das untere Ende des länglichen Gewindeelements (112) ausgebildet wird.

8. Dosiszähler für Inhalatoren nach Anspruch 1, wobei das erste Abdeckelement (126) durchsichtig ist und das zweite Abdeckelement (128) ein Fenster (128a), um die Bewegung des Zeigers (116a) zu sehen, sowie eine lineare Skala angrenzend an das Fenster enthält, um entsprechend der Position des Zeigers die Anzahl der Dosen der Aerosolflüssigkeit anzuzeigen, die in dem Behälter zur Verfügung stehen.

9. Dosiszähler für Inhalatoren nach Anspruch 1, wobei das zweite Abdeckelement (128) Bügel (128b) enthält, die auf einer Rückseite desselben ausgebildet sind, um dieses auf der Außenfläche der Wand (103) des Gehäusekörpers (102) zu stützen, wenn das zweite Abdeckelement auf der Wand über dem ersten Abdeckelement (126) einrastet.

## Revendications

1. Compteur de doses pour inhalateur permettant d'indiquer la quantité de dosage disponible à l'intérieur d'un inhalateur (100), l'inhalateur comprenant une cartouche contenant un fluide d'aérosol et un actionneur comprenant un logement (102) avec une cavité allongée (104) permettant d'y loger la cartouche, le logement ayant une extrémité supérieure et une extrémité inférieure et définissant un axe vertical à travers celui-ci, le logement comprenant un embout (110) s'étendant à partir de l'extrémité inférieure, une tige (110a) s'étendant de façon axiale à partir de l'embout dans la cavité (104) du logement, la tige comportant une cuvette et un passage traversé par un orifice, de sorte que la tige et la cuvette sont configurées pour recevoir une soupape à ressort de la boîte à aérosol et pour distribuer une dose prémesurée d'aérosol dans la bouche d'un utilisateur par la tige et l'orifice, une paroi (103) du logement (102) comprenant une surface extérieure définissant une fente (103a) s'ouvrant dans la cavité allongée (104) du logement ;
ledit compteur de doses pour inhalateur comprenant :
▪ un élément élastique (120) défini par:
◆ un élément central allongé (120a),
◆ des bras (120b) déplaçables de manière élastique s'étendant de part et d'autre de l'élément central allongé (120a),
◆ un élément clé (120c) configuré au bas dudit élément central allongé (120a), un premier nez (120c') intégré audit élément clé s'étendant de manière fonctionnelle vers l'intérieur à partir d'un côté dudit élément central allongé, dans lequel, dans une configuration opérationnelle dudit compteur de doses pour inhalateur, ledit premier nez (120c') s'étend à travers ladite fente (103a) dans la cavité allongée (104) du logement (102) et s'engage avec la tête de la cartouche pour être déplacé à l'intérieur de ladite fente au cas où la cartouche est enfoncée par l'utilisateur ; et
◆ un pousseur (120d) configuré sous l'un desdits bras (120b) et s'étendant depuis ledit élément central allongé (120a), ledit pousseur ayant un deuxième nez de poussée (120e) ;
▪ une formation de roue (118) ayant une formation de roue à rochet (118a) intégrée à ladite formation de roue sur l'un de ses côtés opérationnels et une première formation d'engrenage conique (118b) intégrée à ladite formation de roue sur l'autre côté opérationnel, de sorte que dans la configuration opérationnelle dudit compteur de doses pour inhalateur, ledit deuxième nez de poussée (120e) engage ladite roue à rochet (118a) pour déplacer de manière angulaire ladite roue à rochet, dent par dent, dans une direction ;
▪ un élément fileté allongé (112) ayant une partie filetée hélicoïdale (112a), une deuxième formation d'engrenage conique (124) s'étendant à partir d'une extrémité inférieure opérationnelle dudit élément fileté allongé et configurée pour engager ledit premier engrenage conique (118b) dans la configuration opérationnelle dudit compteur de doses pour inhalateur, dans lequel le rapport d'engrenage entre lesdits premier et deuxième engrenages coniques (118b, 124) est tel que le mouvement d'une dent de ladite roue à rochet (118a) déplace de manière angulaire lesdites première et deuxième roues coniques (118b, 124) d'une dent chacune, provoquant ainsi un mouvement de rotation de l'élément fileté allongé (112), y compris la partie filetée hélicoïdale (112a) de celui-ci, d'un pas ;
▪ une formation filetée (116) configurée pour s'engager dans les filets (112a) dudit élément fileté allongé, et un pointeur (116a) s'étendant à partir de ladite formation filetée, ladite formation filetée pouvant être déplacée le long des filets dudit élément fileté allongé en réponse au mouvement de rotation dudit élément fileté allongé ;
**caractérisé en ce que:**
• un premier élément de couverture (126) comprenant une pluralité de formations formées sur une surface intérieure dudit premier élément de couverture, lesdites formations supportant ledit élément élastique (120), ladite formation de roue (118), ledit élément fileté allongé (112) et ladite formation filetée (116) ;
• ledit premier élément de couverture (126) et ledit élément élastique (120), ladite formation de roue (118), ledit élément fileté allongé (112) et ladite formation filetée (116) assemblés à l'intérieur et soutenus par lesdites formations, étant montés par encliquetage sur ladite surface extérieure de ladite paroi (103) dudit logement (102) dans la configuration opérationnelle dudit compteur de doses pour inhalateur ; et
• un deuxième élément de couverture (128) est encliqueté sur ladite paroi (103) par-dessus ledit premier élément de couverture (126) dans la configuration opérationnelle dudit compteur de doses pour inhalateur ;
dans lequel lesdites formations comprennent:
o une première formation de rail (126a) formée sur ladite surface intérieure dudit premier élément de couverture (126), ladite formation de rail supportant ladite formation filetée (116) et facilitant le déplacement de ladite formation filetée sur celle-ci ;
o un premier support de guidage (126b) formé sur ladite surface intérieure dudit premier élément de couverture (126) à une partie inférieure de celui-ci, ledit premier support de guidage permettant à l'élément clé (120c) comprenant une partie dudit premier nez (120c1) de glisser à l'intérieur dans la configuration opérationnelle dudit compteur de doses ;
o un deuxième support de guidage fermé (126c) formé sur ladite surface intérieure dudit premier élément de couverture (126), ledit deuxième support de guidage fermé permettant à une extrémité supérieure dudit élément central allongé (120a) de glisser à l'intérieur dans la configuration opérationnelle dudit compteur de doses ;
o un troisième support de guidage (126d) formé sur ladite surface intérieure dudit premier élément de couverture (126) dans une partie supérieure de celui-ci, ledit troisième support de guidage accueillant une extrémité supérieure dudit élément fileté allongé (112) et facilitant le mouvement de rotation dudit élément fileté allongé dans la configuration opérationnelle dudit compteur de doses ;
o un quatrième support de guidage (126e) formé sur ladite surface intérieure dudit premier élément de couverture (126), ledit quatrième support de guidage accueillant une partie dudit élément fileté allongé (112) et facilitant le mouvement de rotation dudit élément fileté allongé dans la configuration opérationnelle dudit compteur de doses ; et
o un cinquième support de guidage (126f) formé sur ladite surface intérieure dudit premier élément de couverture (126), ledit cinquième support de guidage accueillant une bague (112b) formée sur ledit élément fileté allongé (112) dans la configuration opérationnelle dudit compteur de doses ;
o un premier tampon (126g) formé sur ladite surface intérieure dudit premier élément de couverture (126) et une surface supérieure dudit cinquième support de guidage (126f) formant un deuxième tampon (126g'), lesdits premier et deuxième tampons facilitant l'appui des extrémités desdits bras (120b) déplaçables de manière élastique, leur déformation élastique et leur glissement dans la configuration opérationnelle dudit compteur de doses ;
o un axe cylindrique (126h) formé sur ladite surface intérieure dudit premier élément de couverture (126) dans sa partie inférieure, ladite formation de roue (118) étant montée de façon rotative sur ledit axe cylindrique dans la configuration opérationnelle dudit compteur de doses ;
o un cliquet (126i) formé de manière angulaire dans une cavité (126f) formée sous ledit cinquième support de guidage (126f), ledit cliquet s'engageant dans ladite roue à rochet (118a) pour empêcher le mouvement inverse de ladite roue à rochet dans la configuration opérationnelle dudit compteur de doses ; et
o une formation cylindrique creuse (126j) et une formation de support (126k).

2. Compteur de doses pour inhalateur selon la revendication 1, dans lequel ladite paroi (103) dudit logement (102) comprend une formation de rail de support (103b), un support de guide de support (103c) et une pluralité de fixations saillantes (103d, 103e) et de supports (103f) formés sur la surface extérieure de ladite paroi.

3. Compteur de doses pour inhalateur selon la revendication 2, dans lequel, dans la configuration opérationnelle du compteur de doses pour inhalateur,
• ladite formation de rail de support (103b) formée sur ladite surface extérieure de ladite paroi (103) est espacée de manière correspondante de ladite première formation de rail (126a) formée sur ladite surface intérieure dudit premier élément de couverture (126) et recouvre partiellement ladite partie filetée hélicoïdale (112a) dudit élément fileté allongé (112) ;
• ledit support de guidage (103c) formé sur ladite surface extérieure de ladite paroi (103) est espacé de manière correspondante dudit cinquième support de guidage (126f) formé sur ladite surface intérieure du premier élément de couverture (126), de sorte que ledit support de guidage (103c) et ledit cinquième support de guidage (126f) enferment ensemble ladite bague (112b) à l'intérieur ; et
• lesdits éléments saillants (103d, 103e) formés sur ladite surface extérieure de ladite paroi (103) sont logés dans ladite formation cylindrique creuse (126j) et dans la formation de support (126k) respectivement.

4. Compteur de doses pour inhalateur selon la revendication 1, dans lequel ladite paroi (103) est une paroi avant du logement (102).

5. Compteur de doses pour inhalateur selon la revendication 1, dans lequel ladite surface intérieure se trouve sur la face arrière du premier élément de couverture (126).

6. Compteur de doses pour inhalateur selon la revendication 1, dans lequel, dans la configuration opérationnelle dudit compteur de doses,
• ladite formation de roue à rochet (118a) est proche de ladite surface intérieure dudit premier élément de couverture (126) et engage ledit deuxième nez de poussée (126e) lorsque ladite formation de roue (118) est montée sur ledit axe cylindrique (126h), et
• ledit premier engrenage conique (118b) est distal par rapport à ladite surface intérieure dudit premier élément de couverture (126) lorsque ladite formation de roue (118) est montée sur ledit axe cylindrique (126h) et engage ledit deuxième engrenage conique (124) dudit élément fileté allongé (112), ledit premier engrenage conique (118b) étant proximal par rapport à ladite surface extérieure de ladite paroi (103).

7. Compteur de doses pour inhalateur selon la revendication 1, dans lequel ladite bague (112b) est formée à côté de l'extrémité inférieure de l'élément fileté allongé (112).

8. Compteur de doses pour inhalateur selon la revendication 1, dans lequel le premier élément de couverture (126) est transparent et le deuxième élément de couverture (128) comprend une fenêtre (128a) permettant de visualiser le mouvement de l'aiguille (116a) et une échelle linéaire adjacente à la fenêtre pour indiquer le nombre de doses de fluide aérosol disponibles dans la boîte correspondant à la position de l'aiguille.

9. Compteur de doses pour inhalateur selon la revendication 1, dans lequel le deuxième élément de couverture (128) comprend des supports (128b) formés sur sa face arrière pour le soutenir sur la surface extérieure de la paroi (103) du logement (102) lorsque le deuxième élément de couverture est encliqueté sur la paroi par-dessus le premier élément de couverture (126).
